# EUROPÄISCHE PATENTANMELDUNG

(11) **EP 0 832 627 A2**
(43) Veröffentlichungstag der Anmeldung: **01.04.1998**
(21) Anmeldenummer: 97115573.4
(22) Anmeldetag: 09.09.1997
(51) Int. Cl.: A61F 13/02

(54) **Pflaster mit holographischer Abbildung**

(30) Priorität: 27.09.1996 DE 19639775
(71) Anmelder: Beiersdorf Aktiengesellschaft, 20245 Hamburg (DE)
(72) Erfinder: Schultz, Günther, 22457 Hamburg (DE); Scheidweiler, Uwe, 24747 Hasloh (DE)

(57) **Zusammenfassung**

Pflaster zum Verkleben auf der Haut, insbesondere zur Abdeckung von kleineren Wunden, bestehend aus einem Trägermaterial, das auf der unteren Seite mit einer hautverträglichen selbstklebenden Schicht versehen ist, dadurch gekennzeichnet, daß auf der oberen Seite des Trägermaterials eine holographische Abbildung vorgesehen ist.

## Beschreibung

Die Erfindung betrifft die Weiterentwicklung bekannter Pflaster.

Die Verwendung von Pflastern zur Abdeckung von Wunden ist allgemein angewandte Praxis. Die Pflaster bestehen aus einem Trägermaterial, das auf einer Seite mit einer selbstklebenden Schicht versehen ist. Auf diese selbstklebende Beschichtung ist in den meisten Fällen eine Wundabdeckung aufgebracht. Um eine einfache Handhabung zu gewährleisten, wird die selbstklebende Beschichtung darüber hinaus mit einer schützenden Schicht aus beispielsweise Folie eingedeckt. Daran anschließend werden einzelne Pflaster aus einer Rolle in unterschiedlichen Formen ausgestanzt und in Papier eingesiegelt. Es handelt sich bei den ausgestanzten Formen um geometrisch regelmäßige Formen wie zum Beispiel Rechtecke oder Kreise.

Bei der Holographie handelt es sich um ein Aufnahmeverfahren, bei dem beliebige Objekte dreidimensional reproduziert werden können (siehe dazu Römpp's Chemie-Lexikon Band 3, 9. Auflage, Georg Thieme Verlag Stuttgart, 1990).
Zur Erzeugung einer Holographie wird das abzubildende Objekt mit monochromatischem und kohärentem Licht beleuchtet. Als Quelle für ein derartiges Licht eignet sich hervorragend ein Laser. Die vom Objekt reflektierte beziehungsweise gestreute Lichtwelle wird mit einem direktem Teilstrahl des Lasers des gleichen Lasers (der sogenannte Referenzstrahl) auf der Photoplatte überlagert. Dabei kommt es zur Interferenz der beiden Wellen, d.h., je nach Wegunterschied zur Verstärkung oder Auslöschung der Wellen. Auf der Photoplatte entsteht ein unregelmäßiges Muster von hellen und dunklen Flecken, Ringen und Wirbeln, das sogenannte Hologramm. Wird die entwickelte Photoplatte wieder mit kohärentem Licht bestrahlt, so ist die von ihr auslaufende Welle in Amplitude und Phase gleich der Objektwelle. Damit entsteht ein virtuelles Bild.

Jeder Bereich des Hologramms enthält, dem Blickwinkel entsprechend, Informationen über das gesamte Objekt.
Bei den bevorzugten Weißlicht-Hologrammen werden Objekt und Referenzwelle aus entgegengesetzter Richtung kommend überlagert. In der relativ dicken lichtempfindlichen Schicht der Photoplatte bilden sich stehende Wellen, also viele übereinanderliegende Hologramme.
Wird eine derartige Schicht wieder mit weißem Licht beleuchtet, so werden an periodisch angeordneten Schichten jeweils Lichtstrahlen reflektiert, die miteinander interferieren können. das betrachtete Weißlicht-Hologramm erscheint je nach Blickwinkel in einer anderen Farbe.

Da die Hologramme nicht kopiert werden können, werden sie heutzutage beispielsweise auf den weit verbreiteten Chipkarten (ec-Karte) eingesetzt. Weiterhin finden sie eine zunehmende Verwendung auf Stickern, Geschenkpapieren und Verpackungen.

Das deutsche Gebrauchsmuster DE G 74 20 413 beschreibt eine Plakatplakette mit einer zwei- oder dreidimensionalen Wiedergabe von mindestens einer Kindern bekannten und vorzugsweise bei diesen beliebten figürlichen Darstellung an ihrer Sichtoberfläche, die dadurch gekennzeichnet ist, daß sie als Wundpflaster mit einer das Trägermaterial für die figürliche Darstellung bildenden und auf der Haut zum Haften bringbare Bereiche aufweisenden Deckschicht aus wundfreundlichem und/oder heilungsförderndem und/oder atmungsaktivem Material ausgebildet ist.
Bei dieser Art von Darstellungen handelt es sich aber stets um einen Aufdruck der herkömmlichen Art. Ein besonderer optischer Effekt ist damit nicht zu erzielen.

Allgemein weisen Pflaster aber überwiegend keinen Aufdruck auf der körperabgewandten Seite des Trägermaterials des Pflasters auf.
Erst seit neuerer Zeit sind vergleichbare Pflaster am Markt anzutreffen. So werden sogenannte Junior-Strips ® mit einem Aufdruck auf einem Pflaster der herkömmlichen Aufmachung vertrieben, das sich insbesondere bei Kindern größter Beliebtheit erfreut. Dieser Aufdruck besteht zumeist aus der Darstellung einer Comicfigur.

Jugendliche und Kinder stehen Pflastern der bekannten Art trotz der unbestrittenen Vorteile bei der Anwendung zur Abdeckung kleinerer Wunden eher ablehnend gegenüber, denn mit dem Pflaster werden negative Erinnerungen wie Schmerz, eventuell Bluten und eine Verletzung verbunden.
Darüber hinaus sehen die herkömmlichen Pflaster unauffällig und damit für Kinder eher unattraktiv aus.

Aufgabe der Erfindung war es, insbesondere für Jugendliche und Kinder ein Pflaster zu schaffen, dem die genannten Personengruppen nicht mehr ablehnend gegenüber stehen, sondern das sie sehr interessant finden, und dies mit äußerst einfachen Mitteln. Im speziellen ist die Erfindung auf die Möglichkeit gerichtet, mit Hilfe der erfindungsgemäßen Pflaster offene Wunden an der Hautoberfläche abdecken zu können, um unerwünschte Folgen wie Infektionen zu unterbinden.

Gelöst wird diese Aufgabe durch ein Pflaster, wie es in den Ansprüchen näher beschrieben ist.

Die Pflaster bestehen aus einem Trägermaterial, bevorzugt einer Polyesterfolie, das auf der oberen Seite eine holographische Abbildung aufweist und auf der unteren Seite mit einer hautverträglichen selbstklebenden Schicht versehen ist.
Neben den genannten Folien können als Trägermaterial aber alle für die Haut, zum Bedrucken und zum Beschichten mit einer selbstklebenden Masse geeignete Materialien Verwendung finden.

In einer bevorzugten Ausführungsform ist das Pflaster mit einer Wundauflage auf der klebenden Seite des Trägermaterials versehen, wobei die Wundauflage kleiner als die Klebfläche ist und in der Mitte des Trägermaterials aufgebracht ist.

Um die Wundauflage gegen Kontamination und Verschmutzung zu schützen, kann die selbstklebend ausgerüstete Seite des Trägermaterials mit mindestens einem abziehbaren Deckblatt als Schutzabdeckung versehen sein.

Um den optischen Eindruck des erfindungsgemäßen Pflasters zu erhöhen, können sowohl die holographische Abbildung auf der Oberseite des Pflasters als auch das Trägermaterial ein- oder mehrfarbig sein.

In einer weiteren bevorzugten Ausführung stimmt die geometrische Form des Trägermaterials im wesentlichen mit den äußeren Konturen der holographischen Abbildung überein.

Als holographische Abbildung ist nahezu jede beliebige Vorlage denkbar. Die Auswahl der Motive kann sich dabei an dem Alter der jeweiligen Zielgruppe für das erfindungsgemäße Pflaster orientieren.
Für Kleinkinder sind holographische Abbildungen von Puppen, Teddys oder ähnlichem vorstellbar, für Kinder bekannte Figuren aus der weiten Wert der Comics, zum Beispiel Asterix oder Obelix, aber auch Figuren aus dem Umkreis von Donald Duck und Micky Maus, oder allgemein dem jeweiligen Zeitgeschmack entsprechende Motive, beispielsweise Dinosaurier, des weiteren einfach Darstellungen von Pflanzen, Tieren oder Menschen. Für Jugendliche sind dann zum Beispiel Pflaster samt dazugehöriger holographischer Abbildung von Markenartikeln (zum Beispiel die typische Coca-Cola-Dose) oder auch Marken (beispielsweise die Symbole der bedeutenden Automobilfirmen).
Diese aufgeführten Beispiele stellen aber nur einen kleinen Ausschnitt aus dem fast unbegrenzten Bereich der Möglichkeiten dar. Dem Fachmann stehen vielfältige Möglichkeiten offen, das erfindungsgemäße Pflaster in seiner Ausführung dem jeweils gewünschten Zweck bzw. der jeweils gewünschten Zielgruppe anzupassen. Die Zahl der Gestaltungsmöglichkeiten für das erfindungsgemäße Pflaster ist durch die Auswahl eines mehrfarbigen Aufdrucks nahezu beliebig erhöhbar. Weitere Ausführungsarten lassen sich durch die Auswahl eines ein- oder mehrfarbig gefärbten Trägermaterials gewinnen, so daß eine gezierte Gestaltung des Pflasters für ein optimales Aussehen möglich ist.

Die äußeren Ausmaße des erfindungsgemäßen Pflasters bewegen sich im Rahmen heute üblicherweise verwendeter Pflaster. In einer bevorzugten Ausführungsform weist die horizontale Achse des Pflasters eine Länge von 10 bis 40 mm auf, die vertikale Achse eine Länge von 40 bis 80 mm.

Bedingt durch die Auswahl der eingesetzten Materialien ist das Pflaster lebensmittelgerecht.

Erfindungsgemäße Pflaster verlieren in den Augen der Jugendlichen ihren eigentlichen Charakter als Pflaster. Sie vermögen den Eindruck eines beliebten Stickers oder gar einer Art Tätowierung hervorzurufen. Die Abneigung der Kinder und Jugendlichen gegen derartige Produkte ist sehr gering, so daß die Verwendung erfindungsgemäßer Pflaster neben dem positiven Einfluß auf den Verlauf der Wundheilung gleichzeitig eine Art Schmuckcharakter erzielt.

Im folgenden soll anhand eines Beispiels der Herstellungsprozeß eines erfindungsgemäßen Pflasters dargelegt werden, ohne in irgendeiner Form einschränkend wirken zu sollen.

### Beispiel

Zur Herstellung der Pflaster wurde zunächst das Trägermaterial, eine 60 µm dicke, transparente Polyethylen-Pflasterfolie, mit einer hautverträglichen Klebstoffschicht auf Basis vernetzter Polyacrylsäure-Derivate beschichtet, mit einer Wundauflage versehen und mit einer die Klebstoffschicht abdeckenden Schutzfolie kaschiert.
Eine Klebemasse des genannten Typs ist in der Schrift DE 27 43 979 C3 dargelegt. Im Heißprägeverfahren (Temperatur von 130 °C) wurden dann die holographischen Abbildungen auf das Trägermaterial aufgetragen, und zwar in einer handelsüblichen Etikettiermaschine vom Typ Gallus ® bei einer Durchlaufgeschwindigkeit von 25 m/min. Die übertragene Schichtdicke der holographischen Abbildung belief sich auf etwas über 0,1 nm.

Die eingesetzte Prägefolie hatte den folgenden Aufbau:
- Trägermaterial aus Polyethylenterephthalat (PET) mit einer Dicke von 12 µm
- 1 µm dünne Wachsschicht unterhalb der Trägerfolie
- Lackschicht
- Aufdampfung einer 0,1 nm dünnen Aluminiumschicht
- Klebebeschichtung

Die Lackschicht selbst setzte sich aus drei Schichten zusammen, nämlich einer Schutzschicht, einem Prägelack und einem Schutzlack, die jeweils eine Dicke von unter 1 µm hatten. Die Lackschicht hatte die Aufgabe, die holographische Abbildung auf dem Pflaster gegen äußere Beschädigung zu schützen.

Die handelsübliche Klebebeschichtung (Hersteller die Firma Kurz), die aus Lacken und Wachsen gebildet wurde, garantierte die Haftung der Aluminiumschicht auf dem Folienträger.
Auf das Trägermaterial des Pflaster wurden beim Heißprägen insgesamt die Lackschicht, die Aluminiumschicht und die Klebebeschichtung übertragen.

Nach dem Aufprägen der holographischen Abbildungen auf die einzelnen Pflaster erfolgte eine Einsiegelung in Papier, um das Pflaster gegen äußere Verschmutzung oder Kontamination zu schützen.

## Patentansprüche

1. Pflaster zum Verkleben auf der Haut, insbesondere zur Abdeckung von kleineren Wunden, bestehend aus einem Trägermaterial, das auf der unteren Seite mit einer hautverträglichen selbstklebenden Schicht versehen ist,
dadurch gekennzeichnet, daß
auf der oberen Seite des Trägermaterials eine holographische Abbildung vorgesehen ist.

2. Pflaster nach Anspruch 1, dadurch gekennzeichnet, daß das Trägermaterial aus einer Polyesterfolie besteht.

3. Pflaster nach Anspruch 1, dadurch gekennzeichnet, daß auf der klebenden Seite des Trägermaterials eine Wundauflage aufgebracht ist, die kleiner als die Klebefläche ist.

4. Pflaster nach Anspruch 1, dadurch gekennzeichnet, daß die Wundauflage in der Mitte des Trägermaterials aufgebracht ist.

5. Pflaster nach Anspruch 1, dadurch gekennzeichnet, daß die selbstklebend ausgerüstete Seite des Trägermaterials mit mindestens einem abziehbaren Deckblatt als Schutzabdeckung versehen ist.

6. Pflaster nach Anspruch 1, dadurch gekennzeichnet, daß die holographische Abbildung und/oder das Trägermaterial ein- oder mehrfarbig sind.

7. Pflaster nach Anspruch 1, dadurch gekennzeichnet, daß die geometrische Form des Trägermaterials mit den äußeren Konturen der holographischen Abbildung im wesentlichen übereinstimmt.
